# EUROPÄISCHE PATENTSCHRIFT

(11) **EP 1 316 530 B1**
(45) Veröffentlichungstag und Bekanntmachung des Hinweises auf die Patenterteilung: **15.12.2004**
(21) Anmeldenummer: 02090410.8
(22) Anmeldetag: 26.04.1999
(51) Int. Cl.: C01B 39/02, A61K 33/08

(54) **Mikronisierte Zoelithe zur Verwendung als pharmazeutisches Mittel**
Micronised zeolites for use as pharmaceutical agents
Zeolithes micronisées destinés à être utilisés comme préparations pharmaceutiques

(43) Veröffentlichungstag der Anmeldung: 04.06.2003
(62) Teilanmeldung aus: 99913546.0
(73) Patentinhaber: Lelas, Tihomir, 10040 Zagreb (HR)
(72) Erfinder: Lelas, Tihomir, 10040 Zagreb (HR)
(74) Vertreter: Omsels, Hermann-Josef

(56) Entgegenhaltungen:
- WO-A-96/34828
- DE-A- 19 755 921
- US-A- 3 323 876
- US-A- 4 173 622
- US-A- 5 704 556

## Beschreibung

Die Erfindung betrifft die Verwendung von mikronisiertem Zeolith als Bestandteil pharmazeutischer Mittel.

Eine Vorrichtung, welche Zeolithe derart zerkleinern kann, dass deren Wirksamkeit verbessert wird, ist bereits aus der DE 197 55 921.2 bekannt. Die dortige Erfindung beschreibt ein Verfahren zur Verbesserung der Wirksamkeit von Wirkstoffen, die mindestens aus Mineralstoffen bestehen, indem diese Wirkstoffe einer tribomechanischen Aktivierung unterzogen werden, bei der die Oberfläche der behandelten Wirkstoffe vergrößert und deren Struktur zur Vergrößerung des chemischen Potentials destabilisiert wird. Die Aktivierung der Mineralstoffe geschieht dadurch, dass in die Integrität der Kristallgitter eingegriffen wird, wodurch sich eine Art Beschädigung ergibt, die sich wiederum in Form einer Aktivierung, beispielsweise auch elektrischer Art, bemerkbar macht. Die DE 197 55 921.2 sieht dabei als vorteilhaft die Behandlung von Zeolithen an, die dort zum heilsamen Verzehr für Menschen beschrieben werden; auch Calcite für den Agrarbereich werden erwähnt. Die durch die aus der DE 197 55 921.2 bekannte Vorrichtung zu erzielende Mikronisierung liegt bei 20 µ pro Teilchen, wobei nur ca. 78 % aller Teilchen diese Größenordnung erreichen.In der DE 197 55 921.2 wird nicht erwähnt oder nahegelegt, warum die Verwendung von mikronisierten Zeolithen als pharmazeutisches Mittel vorteilhaft sein soll.

Ausgehend von diesem Stand der Technik ist es daher Aufgabe der vorliegenden Erfindung, ein pharmazeutisches Mittel zur Verfügung zu stellen, das unter Verwendung von speziell zerkleinertem Zeolith hergestellt wurde.

Gelöst wird diese Aufgabe durch die im Patentanspruch 1 aufgeführten Merkmale, nämlich dadurch, dass mikroniserte Zeolithe mit einem Komgrößendurchmesser von weniger als 0,5 µm zur Herstellung eines pharmazeutischen Mittels verwendet werden, welche durch Rotoren, die aus Scheiben bestehen, auf denen die Ventilatorschaufeln einseitig befestigt angeordnet sind und wobei die Ventilatorschaufeln mit den Kränzen verbunden sind und in korrespondierende Kanäle auf der jeweils gegenüberliegenden Rotorenscheibe durchgreifen, die einen Materialdurchgang unter den Ventilatorschaufeln verhindern, zerkleinert werden. Durch diese Methode der Zerkleinerung wird eine wesentlich effizientere Mikronisierung erreicht unter gleichzeitiger Schonung der Vorrichtung selbst als sie im Stand der Technik bisher bekannt war. Erreicht wird dies dadurch, dass durch die Kanäle die Abnutzung der Ventilatorschaufeln selbst minimiert wird, indem bewusst ein Verbleiben von mikronisiertem Material innerhalb der Kanäle in Kauf genommen wird, was wiederum zu einem erhöhten Widerstand an den Ventilatorschaufeln selbst führt und damit letztlich zu einem erhöhten Grad der Mikronisierung.

Ein nach Anspruch 1 hergestelltes pharmazeutisches Mittel ist erfindungsgemäß zur Herstellung pharmazeutischer Mittel zur Behandlung von Stoffwechsel, Herz-/Kreislauferkrankungen, multipler Sklerose sowie rheumatischer und dermatologischer Erkrankungen vorgesehen.

Die Vorteile der Zerkleinerung lassen sich wie folgt därstellen:

Diese Feinmahlung wird durch kontrollierte Luftströmungen erzielt, die durch die Anwendung von neuartig konstruierten Ventilatorschaufeln hervorgerufen werden.
■ Die Kanäle, in welchen die Ventilatorschaufeln durchgreifen, bilden ein geschlossenes Labyrinthsystem für die Materialverarbeitung, welches die Bewegung des Materials, das der Verarbeitung unterzogen ist, so kontrolliert, dass die Körnchen nicht ohne Schlag- und Reibungswirkung neben den Ventilatorschaufeln unverarbeitet vorbeifliegen, womit die Effizienz der Verarbeitung optimiert wird.
■ Die zu erzielende Mikronisierung liegt bei 98,72 % aller Teilchen unter 4,3 µ. Ein Anteil von 28,36 % aller Teilchen weist sogar einen Durchmesser von unter 0,5 µ auf. Mit keinem herkömmlichen Verfahren oder einer bekannten Vorrichtung konnten derartige Ergebnisse erzielt werden.

Das durch die nachfolgend beschriebene Vorrichtung mikronisierte Material weist vielerlei Vorteile für unterschiedlichste Verwendungsmöglichkeiten auf:

Die beschriebene Zerkleinerung ruft bei mineralischen Rohstoffkomponenten diverse chemische und chemisch-physikalische Veränderungen hervor. Die Effekte, welche durch dynamische Reibungsprozesse entstehen, verleihen diesen Mineralen neue Eigenschaften, die sich bei der Herstellung diverser Produkte technologisch und kommerziell nutzen lassen.

Aus der Gruppe der Zeolithe hat sich das Mineral Heulandit/Klinoptylolith aufgrund seiner Eigenschaften als vorteilhaft herausgestellt, nämlich aufgrund seiner Fähigkeit zur Wasseraufnahme, seiner Selektivität und lonenaustauschkapazität, sowie seiner chemischen Zusammensetzung, welche zeigte, dass dieses Mineral für den menschlichen Genuss absolut unbedenklich ist. Die mineralogischen und chemischen Eigenschaften von Klinoptilolithen wurden untersucht und stellen sich wie folgt dar:

**Tabelle 1**

| **Komponente** | **Anteil [%]** | |
|---|---|---|
| | **von** | **bis** |
| SiO₂ | 61,96 | 67,17 |
| TiO₂ | 0,15 | 0,32 |
| Al₂O₃ | 12,46 | 15,12 |
| Fe₂O | 0,98 | 2,05 |
| MnO | spur | 0,05 |
| MgO | 1,30 | 1,96 |
| CaO | 3,03 | 4,35 |
| Na₂O | 0.70 | 1,11 |
| K₂O | 0,78 | 1,32 |
| H₂O bei 100°C | 4,05 | 4,74 |
| H₂O bei 1000°C | 7,56 | 9,56 |

Der Kalziumgehalt dieses Minerals deutet darauf hin, dass es sich dabei um ein Kalziumzeolith handelt, welches in einer tuffartigen Struktur gebildet ist, d. h. es handelt sich hier um eine Art des Minerals Klinoptilolith mit den Eigenschaften der Heulanditgruppe. Das gemessene Raumgewicht des untersuchten Minerals Klinoptiloithes bewegt sich in dem Bereich von 1,41 bis 1,43 g/cm³. Difraktometrische und thermo-gravimetrische Untersuchungen zeigten, dass in allen untersuchten Mustern ungefähr gleicher Zeolithgehalt besteht. Die Ergebnisse der Röntgenuntersuchungen zeigen die Anwesenheit von folgenden Mineralsorten: Heulandithe (Klinoptilolithe), sowie in weiterer Folge im wesentlichen Quarz, Sand, Plagioklass und in einer geringen Menge auch Biothythe. Die mikroskopische Untersuchung mittels Elektronenmikroskop zeigte, dass die Materialstruktur aus feinen Tuffkömern besteht, welche eine homogene Isotropmasse, praktisch die Zeolithmaterie, darstellt. Der Gehalt dieser Materie bewegt sich grundsätzlich immer im Rahmen zwischen 70 % bis 85 %. Es ist in weiterer Folge die Anwesenheit von eckigen Quarzsegmenten festgestellt worden, sowie plagioklasse Sandkörner, welche in der Regel eine durchschnittliche Korngröße von 60 µ aufweisen. Die Untersuchung des Schmelzpunktes an 10 Mustern hat gezeigt, dass Klinoptilolithe bei einer Temperatur von 1260 - 1280° C schmelzen. Die festgestellte Härte laut Moss beträgt 3 - 3,5. Der Glühverlust beträgt:

**Tabelle 2**

| | | Glühverlust [Gew.-%] |
|---|---|---|
| H₂O | bei 100 °C | 3,34 bis 3,36 |
| H₂O | bei 300 °C | 5,42 bis 5,51 |
| H₂O | bei 500 °C | 2,60 bis 2,64 |
| H₂O | bei 1000 °C | 2,50 bis 2,51 |
| Insgesamt | | 13,86 bis 13,92 |

Ergebnisse der Absorptionsuntersuchungen:

**Tabelle 3**

| Zeitraum | Wasser [Gew.-%] | | Benzol [Gew.-%] | |
|---|---|---|---|---|
| [h] | von | bis | von | bis |
| 1 | 4,61 | 4,62 | 7,64 | 7,65 |
| 2 | 8.74 | 8,75 | 9,33 | 9,33 |
| 3 | 10,75 | 10,77 | 9,45 | 9,47 |
| 4 | 11,10 | 11,11 | 9,51 | 9,54 |
| 17 | 13,44 | 13,45 | 9,54 | 9,54 |

| Weitere Eigenschaften des untersuchten Klinoptitolithes: | | | |
|---|---|---|---|
| 1. | Druckfestigkeit | a)bei trockenem Zustand | max. 426 kg/cm³ |
| | | | min. 361 kg/cm³ |
| | | | mittel 391 kg/cm³ |
| | | b) mit Wasser gesättigten Zustand | |
| | | | max. 360 kg/cm³ |
| | | | min. 253 kg/ cm³ |
| | | | mittel 292 kg/cm³ |
| 2. | Wasseraufnahme | | 23,35 Gew.-% |
| 3. | Raumgewicht | | 1,37 g/cm³ |

Die Untersuchungsergebnisse der elektrischen Leitfähigkeit zeigten, dass die erfindungsgemäß behandelten Zeolithe bedeutend mehr Wasserstoffionen zu binden vermögen als die unbehandelten Zeolithe. Dies ist die unmittelbare Folge der Unterschiede in der Kristallstruktur der untersuchten Zeolithe, die durch die Feinmahlnung und Mikronisierung zustandegekommen sind. Die folgende Tabelle zeigt Beispiele für die Messungen der Leitfähigkeit und des pH der Suspension bei nicht behandelten und behandelten Zeolithen:

**Tabelle 4**

| Mischzeit der Suspension ^{a} | Konzentration an Zeolith (mg/ml H₂O) | pH^{b} (unaktiviertes Zeoith) | pH^{b} (erfindungsgemäß behandeltes Zeolith) | Leitfähigkeit^{c} (unaktiviertes Zeolith) µS/cm | Leitfähigkeit^{c} (erfindungsgemäß behandeltes Zeolith) µS/cm |
|---|---|---|---|---|---|
| 2 Std. | 20 | 7,35 | 7,48 | 157 | 147 |
| 2 Std. | 40 | 6,45 | 7,22 | 198 | 180 |
| 2 Std. | 60 | 6,81 | 7,35 | 307 | 280 |
| 20 Std. | 40 | 6,67 | 6,90 | 259 | 218 |
| 20 Std. | 80 | 6,96 | 7,40 | | |
| 48 Std. | 80 | 6,87 | 7,44 | | |

| | | | | | |
|---|---|---|---|---|---|
| ^{a} mit Magnetrührer, 70 - 100 RPM; | | | | | |
| ^{b} pH Messung unmittelbar nach der Zentrifugation (15 Minuten auf 7000 RPM), | | | | | |
| ^{c} Leitfähigkeitsmessung im Überstand unmittelbar nach der Zentrifugation (15 Minuten auf 7000 RPM), Leitfähigkeit des redestillierten Wassers beträgt 1.8 µS/cm | | | | | |

Die Ergebnis ist, dass der pH-Wert der unbehandelten zu den behandelten Zeolithe steigt, während die Leitfähigkeit abnimmt. Dies führt zu der überraschenden Erkenntnis, dass das behandelte Zeolith aufgrund der sinkenden Leitfähigkeit bestens dazu prädestiniert ist, als natürliches und physiologisch wirksames Puffermaterial eingesetzt zu werden, denn das Puffervermögen des behandelten Zeolithpulvers und die damit verbundene Beständigkeit des pH-Wertes führt dazu, dass das behandelte Material, z.B. auf seinem Weg durch den Verdauungstrakt, von der Magensäure nicht zur Gänze, sondern nur geringfügig gesättigt wird. Somit bleibt das behandelte Zeolith auch im Dünndarm aktiv, was letztlich ein wünschenswertes Ergebnis ist..

Die Interaktion von behandelten Zeolithmaterial bei dem Reaktionsmodell der Oxidation von L-Askorbaten/Askorbinsäure mit Nitrobenzol^{(a)} beträgt:

**Tabelle 5**

| Lösungsmittel | Nitrobenzol/M | L-Askorbinsäure/Askorbat_{TOT} /M | Erfindungsgemäß behandeltes Zeolith [mg/ml] | K_{obs}/s⁻¹ ^{(b)} | T/°C |
|---|---|---|---|---|---|
| Wasser, | 3,0 x 10⁻⁴ | 3,0 x 10⁻⁴ | - | 0,1599 (0,0112) | 10 |
| pH = 4,22 | 3,0 x 10⁻⁴ | 3,0 x 10⁻⁴ | 0,375 | | |
| Wasser, | 1,2 x 10⁻⁴ | 1,2 x 10⁻³ | - | 0,6148 (0,0309) | 25 |
| pH = 3,75 | 1,2 x 10⁻⁴ | 1,2 x 10⁻³ | 0,504 | 0,7537 (0,0112) | 25 |
| | 1,2 x 10⁻⁴ | 1,2 x 10⁻³ | 0,500 | 0,6673 (0,0144) | 25 |
| | | | (nichtaktiviertes | | |
| | | | Zeolith) | | |
| Wasser, | 1,2 x 10⁻⁴ | 1,8 x 10⁻³ | - | 0,9374 (0,0287) | 25 |
| pH = 6,63 | 1,2 x 10⁻⁴ | 1,8 x 10⁻³ | 0,502 | 0,7473 (0,0845) | 25 |
| | 1,2 x 10⁻⁴ | 1,8 x 10⁻³ | 0,500 | 0,7964 (0,0134) | 25 |
| | | | (nichtaktiviertes | | |
| | | | Zeolith) | | |
| Dioxan/Wasser | 1,2 x 10⁻⁴ | 1,8 x 10⁻³ | - | 0,2981 (0,0246) | 25 |
| 50/50 % v/v | 1,2 x 10⁻⁴ | 1,8 x 10⁻³ | 0,508 | 0,2247 (0,0293) | 25 |
| pH = 6,66 | | | | | |
| Dioxan/Wasser | 1,2 x 10⁻⁴ | 1,8 x 10⁻³ | - | 0,6675 (0,0111) | 25 |
| 50/50 % v/v | 1,2 x 10⁻⁴ | 1,8 x 10⁻³ | 0,505 | 0,6021 (0,0071) | 25 |
| 0,0784 M | | | | | |
| KH₂PO₄+NaOH | | | | | |
| pH = 6,50 | | | | | |
| Wasser 0,0784 M | 1,2 x 10⁻⁴ | 1,8 x 10⁻³ | | 1,8670 (0,0613) | 25 |
| KH₂PO₄+NaOH | 1,2 x 10⁻⁴ | 1,8 x 10⁻³ | 0,502 | 1,8732 (0,0133) | 25 |
| pH = 6,50 | | | | | |

| | | | | | |
|---|---|---|---|---|---|
| ^{a} Modellreaktion nach S. Ursic et al. *New J. Chem.* 1998, 221. Mit erweiterten Untersuchungen der Interaktion von erfindungsgemäß behandeltem Zeolith. ^{b} Mittelwert von 3-5 Messungen | | | | | |

Überraschende Erkenntnisse und damit Vorteile lassen sich wie folgt darstellen:
■ Veränderung des pH-Wertes auf dem zellulärem Niveau, wie z. B. die Entsäuerung von Tumorzellen, welche grundsätzlich einen niedrigen pH-Wert aufweisen; dadurch werden die Antitumorwirkungen verursacht.
■ Selektive Proliferation von Aminosäuren, Peptiden, Oligonukleotiden usw.;
■ Veränderung des lonenaustausches auf den Zellmembranen;
■ Interaktion der Zellrezeptoren und somit Beeinflussung der Zellprozesse;
■ Stärkung der Abwehrkraft des Organismus infolge vorübergehender oder vollkommener Blockade der Carbo-Kationen und freien Radikale;
■ Transport von bioaktiven Molekülen (z. B. Zusammenwirkung mit Silibin (einem Pflanzenextrakt, wie auch Rosskastanienextrakt, Gin Seng und Petersilienextrakt) und/oder Askorbinsäure).
■ Aktivierung und Optimierung der Aktivität der Kalium/Natrium-Pumpe auf der Zellmembran; dies führt zu einer Gesundung und Optimierung des Stoffwechselvorgangs der Zellen.
■ Transport von Blutzucker vom Blut in die Zelle ohne Verwendung von Insulin.

Das erfindungsgemäß behandelte Zeolith wurde versuchsweise insgesamt 600 Personen oral und lokal verabreicht. Dabei wurden folgende medizinisch relevante Auswirkungen festgestellt, für die das erfindungsgemäß behandelte Material vorteilhaft ist:
a) Krebserkrankungen
   ■ Hautkrebs
   ■ Lymphdrüsenkrebs
   ■ Knochenkrebs
   ■ Brustkrebs
   ■ Eierstockkrebs
   ■ Gebärmutterhalskrebs
   ■ Prostatakrebs
   ■ Hodenkrebs
   ■ Dünn- und Dickdarmkrebs
   ■ Leber-, Milz-, Gallen-, Bauchspeichelkrebs
   ■ Lungen-, Bronchienkrebs
   ■ Magenkrebs
   ■ Gehirntumore und andere kanzerogene Erkrankungen
b) Herz- und Kreislauferkrankungen
   ■ Funktionsstabilisierung und Optimierung des Gefäßsystems
   ■ Verminderung der Venendurchlässigkeit
   ■ Elastazirierung von erweiterten Venen und Rückbildung von Hämorrhoiden
   ■ Verschwinden erweiterter Kapillaren
   ■ Stärkung der Herzmuskeln
   ■ Stabilisierung des Blut- und Herzdrucks
c) Reduzierung des Blutfettes
   ■ allmähliche Stabilisierung der Blutfettwerte (Chotesterol, Triglyzeride)
   ■ Globale Veränderung des Blutbildes in Richtung optimaler Werte
d) Stabilisierung des Stoffwechsels und des Verdauungssystems
   ■ Optimierung des Stuhlgangs
   ■ Sanierung von Gastritis, Magengeschwür und Zwölffingerdarmgeschwür
e) Heilung von rheumatischen Erkrankungen
   ■ Ischias
   ■ Diskopathie
   ■ Spondilose
   ■ rheumatische Athritis
   ■ Gicht usw.
f) Optimierung der Nierenfunktionen
   ■ Diurethische Wirkung
   ■ Beseitigung von Entzündungen (Nefritis)
   ■ Verbesserung der Filterfunktionen
g) Bekämpfung von Hautkrankheiten
   ■ Seborrhoe
   ■ Dermatitis
   ■ Herpes Simplex und Herpes Zoster
   ■ Psoiasis
h) Diabetes mellitus
   ■ Absenkung des Zuckemiveaus im Blut
   ■ Beseitigung von Beschwerden bei peripherer Zirkulation
i) Endokryne Drüsen
   ■ Optimierung des Hormonausstoßes
   ■ Korrekturen bei unvollständiger Funktionsfähigkeit der Drüsen
j) Heilung bei Wunden und Verbrennungen
   ■ Offene Beine (Ulzera Cruris)
   ■ Dekubitus
   ■ Brandwunden, schmerzstillend und desinfizierend
   ■ Schnittwunden, schmerzstillend und desinfizierend
k) Parodontose
   ■ Eliminierung von Mikroorganismen aus dem Zahnfleisch
   ■ Verschließung und Heilung von offenen Wunden im Mundbereich
l) Beseitigung neuropsychiatrischer Erkrankungen
   ■ Epileptische Erkrankungen
   ■ Schizophrenie
   ■ Alzheimersche Erkrankungen
   ■ Parkinsonsche Erkrankungen
   ■ Neurosen und Depressionen
   ■ Allgemeine Stimmungsverbesserungen
m) Pilzerkrankungen
   ■ antimykotische Wirkung auf der Haut und Schleimhaut (ausgeschlossen Augen)
   ■ antimykotische Wirkung bei den inneren Organen
n) gynäkologische Erkrankungen
   ■ Papiloma Virus (HPW 31)
   ■ Chlamydia
   ■ Verbesserung des Reinheitsgrades des vaginalen Bereiches

Die Testpatienten mit den oben angeführten Indikationen haben das behandelte Material in Pulverform vermischt mit Wasser eingenommen. Die Dosierung betrug zwischen 1 g und 12 g täglich; die Frequenz der Einnahme betrug 3 bis 12 mal pro Tag. Die Dosierung betrug 0,5 g bis 1 g pro Einnahme mit einem Abstand von 1 - 6 Stunden zwischen der Verabreichung.

Verfolgung des Krankheitsablaufs bei Patienten, bei denen die Therapie mit dynamisch feingemahlenen und mikronisierten Zeolithen durchgeführt wurde:

### 1.1 Patienten mit kanzerogenen Erkrankungen

Im Rahmen einer Testreihe wurden in einer Zeitspanne von 5 Monaten 280 Patienten behandelt.

Zerebrale Tumore wurden bei 21 Patienten, die sich in der Terminalphase befanden, verfolgt. Die Patienten waren im schlechten Allgemeinzustand, unbeweglich und bekamen nur eine symptomatische Therapie, d. h. sie bekamen nur schmerzstillende, antidepressive Mittel. 3 - 4 Wochen nach Anfang der Einnahme des aufbereiteten Materials, haben sich deutliche Verbesserungen des allgemeinen Zustands gezeigt. Die Patienten hatten keine Symptome epileptischer Anfälle mehr; sie sind wieder beweglich geworden und einige waren fähig selbst zu lesen, sich zu waschen und normal zu kommunizieren. Die Verbesserung des Allgemeinzustands setzte sich fort, so dass nach 5 Monaten 14 Patienten keine Anzeichen mehr von Krebserkrankungen hatten.

Bei 40 Patienten mit primären Lungentumoren, die sich in der terminalen Phase befanden, wurde der Allgemeinzustand in der 3. und 4. Woche nach dem Einnahmebeginn deutlich verbessert. Die Schmerzen wurden deutlich gemindert, die Respiration und Beweglichkeit wurden beschwerdenfrei. Bei dieser Gruppe wurde nur ein einziger Patient aufgrund eines invasivkahektischen Anfalls verloren.

Ferner wurden 53 Patienten mit terminalen Krebserkrankungen des Verdauungstrakts mit ausgesprochen kahektischen Zustand klinisch verfolgt. Bei diesen Patienten trat die positive Wirkung etwas später ein (Woche 5 bis 7), wobei 4 Patienten in der Anfangsphase der Einnahme verstorben sind. Der Rest der Patienten hat sich gut erholt und nach 5 Monaten hatten nur wenige die Reste der Krankheitssymptome zu verzeichnen.

Bei mehr als 150 Patienten wurde die positive Wirkung des behandelten Materials in Zusammenwirkung mit klassischen Methoden (Chemotherapie, radiologische Therapie) deutlich festgestellt. Die negativen Auswirkungen der klassischen Methoden wurden deutlich verringert.

### 1.2 Lebererkrankungen, Hepatitiszirrhose

Bei 20 Patienten mit chronischen Hepatitis-Viren zeigte bereits eine täglich geringe Dose von 2 - 3 g des behandelten Materials, eine deutliche Verbesserung des Allgemeinzustands. Bereits 2 Wochen nach Anfang der Einnahme waren Müdigkeit und Blähungen nicht mehr vorhanden. In 30 Tagen nach Anfang der Therapie wurden Transaminasen im Blut (AST, HLT, GGT, AP) und Bilirubin vermindert. Die Marker von Hepatitis, DNA und RNA zeigten virusnegative Ergebnisse.

Bei dekompensierten Zirrhosen wurde bereits nach 7 Tagen eine Verbesserung des allgemeinen Zustandes und der Rückgang von Ascites beobachtet.

### 1.3 Diabetes mellitus

Bei 24 Patienten mit insulinunabhängigem Diabetes wurde eine deutliche Glukoseverminderung und Stabilisierung im Blut bereits nach wenigen Tagen der Einnahme festgestellt. Es wurden bei mehreren Patienten daneben anderen therapeutische Mittel abgesetzt, ohne dass dies zu nachteiligen Ergebnissen führte.

### 1.4 Neurodegenerative Erkrankungen

Sehr positive Auswirkungen wurden bei Multipler Sklerose (13 Patienten) in den Anfangsphasen der Erkrankung beobachtet, während bei Patienten in den Terminalphasen (9 Patienten) die weitere Entwicklung der Krankheit gestoppt wurde. Sie zeigten jedoch keine deutliche Besserung des Allgemeinzustandes.

Gute Ergebnisse wurden auch bei Neurodermitis (7 Patienten), muskulären Dystrophien (4 Patienten), Parkinsonschen Krankheiten (5 Patienten), Alzheimerschen Krankheiten (7 Patienten), Arteriosklerose (6 Patienten) und anderen Krankheiten festgestellt.

### 1.5 Andere Krankheiten

Die Einnahme des dynamisch feingemahlenen und mikronisierten Zeoliths hat auch gute Eigenschaften bei der Symptomminderung bei chronischer Arthritis, insbesondere rheumatischer Arthritis, gezeigt. Es wurden auch Erfolge bei dermatologischen Erkrankungen wie z. B. Psoriasis, Lupus Erritematodesa, Wunden- und Verbrennungsheilung beobachtet.

### 1.6 wirksame Zusätze

Die Vermischung des dynamisch feingemahlenen und mikronisierten Zeoliths mit anderen Mitteln erbringt zusätzlich vorteilhafte Ergebnisse, die teilweise sogar zu einer Verstärkung der Wirkung führten. Nachfolgende Mischungen haben sich als vorteilhaft für bestimmte Indikationen erwiesen:
■ mit Gelee Royal (Bienenköniginfutter) für die Vitalitätsverbesserung
■ mit Knoblauchextrakt für die Absenkung des Blutdrucks
■ mit Baldrianextrakt für die Beruhigung
■ mit fein gemahlener Haselnuss für die Bekämpfung rheumatischer Erkrankungen
■ mit Rosskastanienextrakt für die Elastizierung erweiterter Venen
■ mit Petersilienextrakt für die Verbesserung von Diurethik
■ mit Fruchtzucker für die Verbesserung der Muskelresorption
■ mit Sylimarin/Sylibin für die Beseitigung von Leberbeschwerden
■ mit Proteinen für die antikahektische Wirkung

Eine Vorrichtung, welche zur Zerkleinerung des Zeoliths verwendet wird und zu den vorteilhaften Eigenschaften des zerkleinerten Materials führt wird nachfolgend beschrieben. Es zeigt:
- **Figur 1**: eine schematische Ansicht der erfindungsgemäßen Vorrichtung;
- **Figur 2**: einen vertikalen Schnitt durch die Vorrichtung;
- **Figur 3**: eine Rotorenscheibe;
- **Figur 3a**: einen vertikalen Schnitt durch einen Ausschnitt der zusammengesetzten Vorrichtung,
- **Figur 3b**: eine Ausschnittvergrößerung entlang eines Schnittes A-A gemäß der Fig. 3a;
- **Figur 4**: die schematische Andeutung der Luftströme entlang der Ventilatorschaufeln;
- **Figur 5**: die schematische Andeutung des zerkleinerten Materials entlang der Ventilatorschaufeln;
- **Figur 6**: eine Ventilatorschaufel mit Stift/Kanal im Detail und in mehreren Ansichten (**6a, 6b, 6c**);
- **Figur 7**: schematische Darstellung einer Segmenten-Ventilatorschaufel;
- **Figur 7a**: Ausschnitt entlang eines Schnittes A-A gemäß Figur 7.

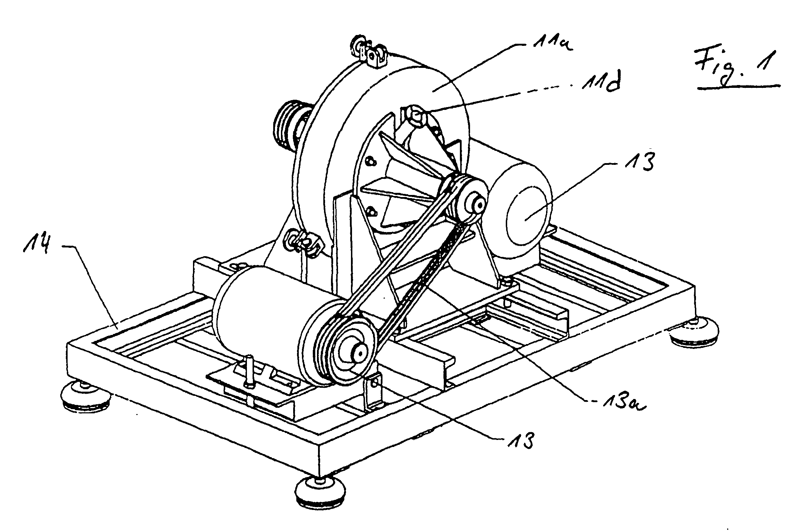

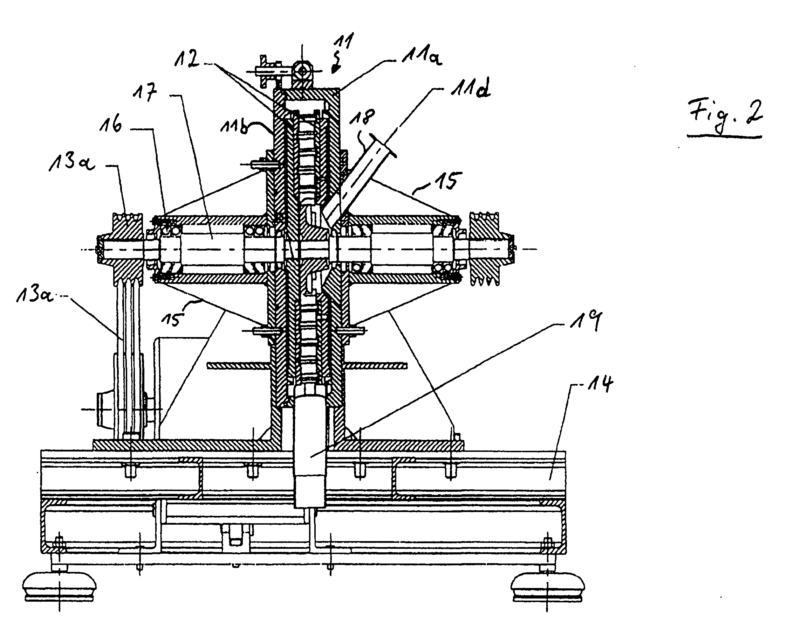

Die Figur 1 zeigt eine Vorrichtung 10 für die Feinmahlung und Mikronisierung, sowie das Homogenisieren von diversen festen und flüssigen Rohstoffkomponenten. Das Prinzip sieht derart aus, daß das Ausgangsmaterial durch die Mitte der Rotoren in den Verarbeitungsraum der Vorrichtung eingesaugt wird. Der Eintritt wird durch die Einwirkung von zentrifugalen Kräften in den Raum zwischen den Ventilatorschaufeln begünstigt und wird aufgrund der dort herrschenden Luftströme beschleunigt; so daß das Material mit dem bereits verarbeiteten Material kollidiert. Das Ausgangsmaterial wechselt die Bewegungsrichtung in sehr kurzen Intervallen; infolgedessen wird es zerkleinert und mikronisiert.

Die Vorrichtung 10 besteht aus einem aufklappbaren Gehäuse 11 mit einem Materialeinfuhrschacht 11d, in welchem sich zwei Rotorenscheiben 12 befinden, die gegeneinander gestellt sind und mittels entsprechender Motoren 13 über Riemen 13a gegenläufig betrieben werden, so daß sie sich mit gleicher Winkelgeschwindigkeit drehen. Das Gehäuse 11 und die Motoren 13 sind auf dem Fundament 14 befestigt und bilden eine unabhängige Einheit.

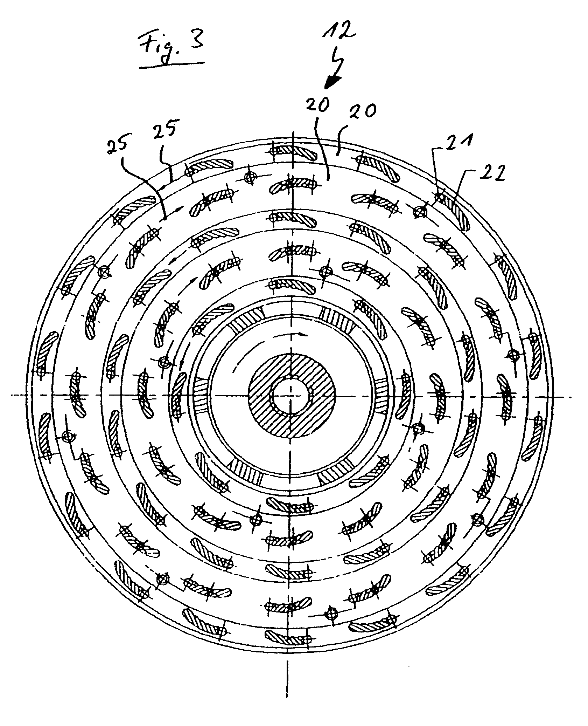

Figur 2 zeigt, daß das Gehäuse 11 aus zwei Teilen aufgebaut ist: eine Gehäuseseite 11a für die Materialeinfuhr und eine weitere Gehäuseseite 11b mit Einfuhrschacht 11 c. Diese zwei Seiten 11 a, 11 b sind miteinander verschraubt. Auf beiden Seiten des Gehäuses 11 befinden sich die Träger 15, in welche die Lager 16 und Reckstangen 17 eingebaut sind. Auf der Seite 11a für die Materialeinfuhr befindet sich ein Rohr 18 für die geregelte Einfuhr des Materials; auf der unteren Seite befindet sich eine Öffnung 19 für den Ausstoß des fertigen Materials.

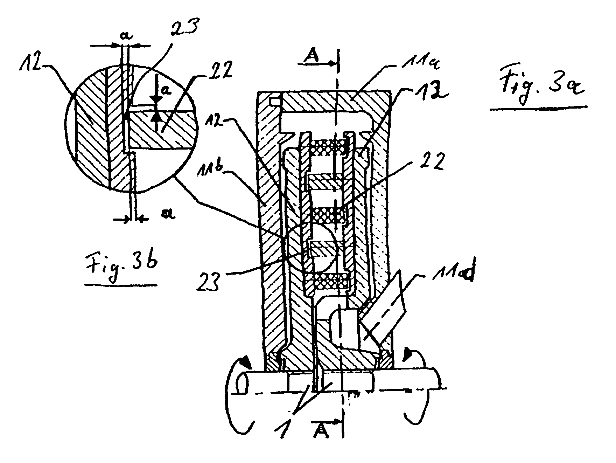

Figur 3, 4 und 5 zeigt, daß auf den Rotorenscheiben 12 mehrere konzentrische Kränze 20 mit den Stiften 21 und Ventilatorschaufeln 22 angeordnet sind, welche so konstruiert und ausgerichtet sind, daß sie berührungsfrei, während sie gegenläufig drehen, nebeneinander vorbeilaufen können - angedeutet durch die Drehrichtung 25. Minimal sind zwei Kränze erforderlich, die von den zwei Rotoren angetrieben werden. Die Aufgabe der Schlagstifte 21 und der Ventilatorschaufeln 22 ist die Erzeugung von turbulenten Luftströmungen für die Beschleunigung des verarbeiteten Materials, so daß Stöße und Reibung unter den Körnchen bei bestimmten Winkeln unter dynamischen Bedingungen hervorrufen werden. Die Kanäle 23 auf den Scheiben verhindern den Materialdurchgang unter den Ventilatorschaufeln 22, vgl. Figur 3 a und 3b.

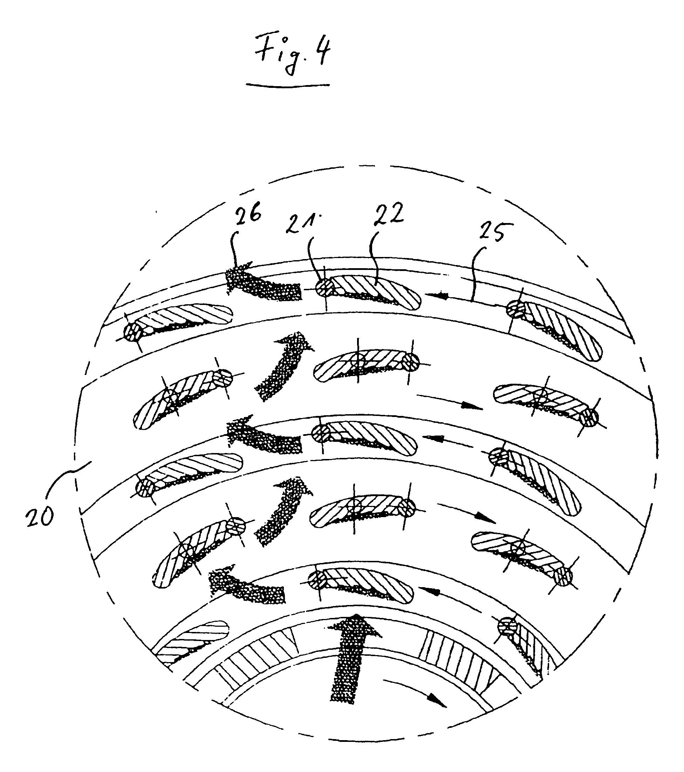

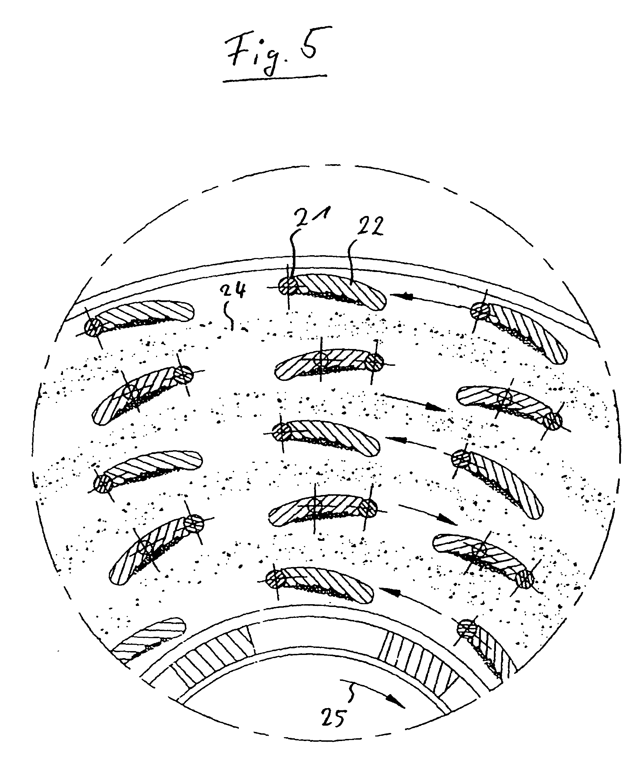

Das Ausgangsgranulat (nicht dargestellt) wird, durch den Zentralteil 18 des Motorensystems durch Einsaugung eingebracht, durch die Luftströme 26 beschleunigt und so gesteuert, daß die Körnchen infolge mehrmaliger Bewegungsrichtung untereinander kollidieren und sich in sehr kurzen Zeitintervallen aneinander reiben. Dabei berühren sich die Arbeitswerkzeuge und andere Teile der Vorrichtung nicht oder nur geringfügig - aber es kommt auf keinen Fall zu Zerstörungen der Werkzeuge. Es entsteht eine Interaktion unter den Körnchen mit solchem Ausmaß, dass bei den Körnchen die innere Energie ausgetauscht wird, da die Zusammenstöße unplastisch sind (vgl. Fig. 4 und 5).

Die Effekte, die Folge der Zusammenstöße der Körnchen sowie Folge der relativen Bewegung der Oberfläche eines Körnchen über der Oberfläche von einem anderen Körnchen (mechanische Reibung) sind, werden verstärkt durch diejenigen Effekte, die infolge plötzlicher Richtungswechsel der Körnchenbewegung entstehen, so dass die Energie der Beschleunigung und relativer Bewegung der Körnchen in Energie der Deformierung, sowie in die Energie der molekularen Bewegung umgewandelt wird. Während der Zusammenstöße und der Reibung der Körnchen, welche dem Verkleinerungsprozess in sehr kurzen Zeitintervallen (10⁻⁵ bis 10⁻⁶ s) unterzogen sind, entsteht eine bedeutende Veränderung ihrer Geometrie bzw. Form und Größe. Durch die relative Bewegung eines Körnchens über der Oberfläche eines anderen Körnchens, entstehen Schäden und Deformierungen der Körnchenoberfläche, sowie der Materialschichten, welche sich unmittelbar unter der Kornoberfläche befinden. Dadurch wird die Struktur des Kristallgitters auf der Oberfläche zerstört oder geschädigt, so dass teilweise die Kristallform in eine amorphe Phase umgewandelt wird, mit dem Ergebnis, dass die physikalischen und physikalisch-chemischen, sowie energetischen Eigenschaften des verarbeitenden Materials verändert werden. Bei solchen Verarbeitungen von Rohstoffkomponenten organischen Ursprungs zerreißen beispielsweise Zellulosefasern und große Molekülen werden mithin zu kleineren Molekülen, wobei diverse Veränderungen chemischer Zusammensetzung bei dem verarbeiteten Material hervorgerufen werden, sowie auch physikalische Veränderungen, die bedeutend für ihre weitere Materialverarbeitungen und/oder Aufbereitung, aber auch für die Wirksamkeit sind.

Neben der Veränderung der Eigenschaften des Materials, welches mit den beschriebenen Verfahren verarbeitet wird, wird dieses infolge von mechanischen Beanspruchungen fein gemahlen und mikronisiert und die Veränderung der granulometrischen Zusammensetzung des Materials ist von der Granulometrie der Ausgangskörner, sowie vom Niveau der Kömchenbeschleunigung, der geplanten Winkel der Zusammenstöße und gegenseitigen Reibung, sowie die geplanten Anzahl der Zusammenstöße abhängig. Folgende Parameter für die Feinmahlung und Mikronisierung mit der erfindungsgemäßen Vorrichtung stellen eine optimale Konfiguration dar, so dass diese Parameter bevorzugt sind:
■ Granulation des Ausgangskorns < 4,0 mm
■ Anzahl der Gesamtzahl der Kränze/Kranzreihen auf den Rotorenscheiben: 5
■ Durchmesser der Rotoren 500 mm
■ Drehzahl der Rotoren 3600 /Min.
■ Kapazität der Vorrichtung 300Kg/Std.

Verglichen mit den Materialien, welche auf herkömmliche technische Weise (vgl. DE 197 55 921.2) feingemahlen und mikronisiert werden, zeigt das Material, welches durch die erfindungsgemäße Vorrichtung feingemahlen und mikronisiert wurde, eine erhöhte freie Energie und Reaktionsfähigkeit.

Die Innovation liegt mithin in der Konstruktion (Form, Verzahnung, Neigung) und Austauschbarkeit von Stiften und Ventilatorschaufeln auf die konstruierten Kanäle, die sich auf der Rotorenscheibe befinden, sowie in der Auswahl des Materials für die Ausarbeitung von Ventilatorschaufeln. Die Rotationsscheiben der Vorrichtung rotieren mit der gleichen Winkelgeschwindigkeit, bewegen sich jedoch gegenläufig. Das Ausgangsmaterial wird durch das Einsaugrohr 18 in den Zentralteil der rotierenden Scheibe eingeführt, aufgrund von Zentrifugalkräften werden die Körnchen der Rohstoffkomponente in Richtung des äußeren Randes des Gehäuses beschleunigt. Die Körnchen schlagen auf die Kränze 20 der Ventilatorschaufeln 22, welche sich in Gegenrichtung drehen. Sie wechseln die Bewegungsrichtung aufgrund der Richtungswechsel der Ventilatorschaufeln. Ferner schlagen und reiben sich die Körnchen untereinander, gehen nachfolgend in einen weiteren Kranz 20 mit Ventilatorschaufeln 22 über, wechseln wieder die Richtung der Bewegung im Einklang mit dem Richtungswechsel der rotierenden Scheibe, solange bis sie das Schaufelsystem verlassen. Am Ende der Bearbeitung in der erfindungsgemäßen Vorrichtung schlagen die Körnchen auf der Wand des Gehäuses auf und werden durch die Ausgangsöffnung 19 abtransportiert.

Figur 3a und 3b zeigt, dass die Kanäle 23 auf den Scheiben 12, in welche die Ventilatorschaufeln 22 durchgreifen, den Materialdurchgang unter den Ventilatorschaufeln 22 verhindern. Deren Form wird gemäß den Eigenschaften der jeweils zu verarbeitenden Rohstoffkomponenten (Materials) - also Granulation des Ausgangsmaterials, seine Feuchtigkeit, Härte, Ursprung, chemische Zusammensetzung und ähnliches - definiert. Wenn z. B. die Eingangsgranulation des Materials < 1 mm beträgt, bedeutet dies, dass der minimale Abstand zwischen den Ventilatorschaufeln und den Kanälen auf den Scheiben größer als 1 mm sein soll, um überhaupt das Durchgehen des Materials zu ermöglichen. Bei der Ausarbeitung und der Montage der Scheiben soll zufriedenstellende Parallelität verwirklicht werden, um den Ventilatorschaufeln das Durchgreifen in die Kanäle zu ermöglichen, denn die Durchmesser der Scheiben sind relativ groß (500 mm). Vorteile gegenüber der in der DE 197 55 921.1 beschriebenen Vorrichtung liegen darin, dass die Ventilatorschaufeln aufgrund ihrer Form, Neigung und Verzahnung bei dem Prozess der Feinmahlung und Mikronisierung mit einer feinen Schicht des verarbeiteten Materials belegt werden und auf diese Weise von der Schlag- und Reibungswirkung des Eingangsmaterials geschützt werden. Die Abnutzung der Oberfläche der Ventilatorschaufeln wird somit minimiert und die Lebensdauer bedeutend verlängert. Ferner wird die energetische Ladung des Materials, welches behandelt wird, angehoben, wenn die Körnchen untereinander kollidieren und nicht mit den Teilen der Ventilatorschaufeln. Die technologischen Parameter, wie die Anzahl der Ventilatorschaufeln, ihre Neigung, die Form der Schaufelverzahnung, Anzahl der Ventilatorkränze, Winkelgeschwindigkeit der Scheiben, definieren die späteren Eigenschaften des verarbeiteten Materials. Mit der Kombination der angeführten Parameter wird es möglich, die Ergebnisse und Effekte zu programmieren.

In der Figur 3b sind weiter Scheibenkanäle (23) näher dargestellt. In den Rotorenscheiben (12) befinden sich an denjenigen Orten, die mit der Kranzreihe (20) des gegenüberliegenden Rotors (13) korrespondieren, Ausnehmungen (20a; siehe Fig. 3a, 3b), innerhalb derer die Ventilatorschaufeln (22) des gegenüberliegenden Kranzes (20) laufen. Die Kanäle (23) decken sich gegenseitig mit der Länge a, welche 2 - 5 mm beträgt. Sie bilden quasi ein Labyrinth, welches den Durchgang des Materials unter den Ventilatorschaufeln verhindert. Durch die Entstehung des Labyrinthes verstärkt sich der Widerstand für die Strömung unter den Ventilatorschaufeln. Somit wird erreicht, dass sich die Körnchen des Ausgangsmaterials durch die Hauptströmung zwischen den Ventilatorschaufeln bewegen. Die Form der Kanäle wird gemäß technisch-technologischer Eigenschaften der Rohstoffkomponenten des verarbeiteten Materials definiert (Granulation des Ausgangsmaterials, seine Feuchtigkeit, Härte, Ursprung, chemische Zusammensetzung und ähnliches). Wenn es die Scheibenkanäle nicht geben würde, würde sich das Ausgangsmaterial aufgrund der Zentrifugalkräfte so bewegen, dass es vom Zentrum bis zu der Peripherie der Scheiben an den Ventilatorschaufeln und der Scheibe vorbeigeht. Bei der Ausgangsgranulation des Materials von 0 - 1 mm muss die Entfernung zwischen den Ventilatorschaufeln und den Kanälen größer als 1 mm sein, um den Durchgang des Materials überhaupt zu ermöglichen, vorteilhaft hat sich eine Entfernung von 2 mm genauso wie für die Verdeckung a erwiesen.

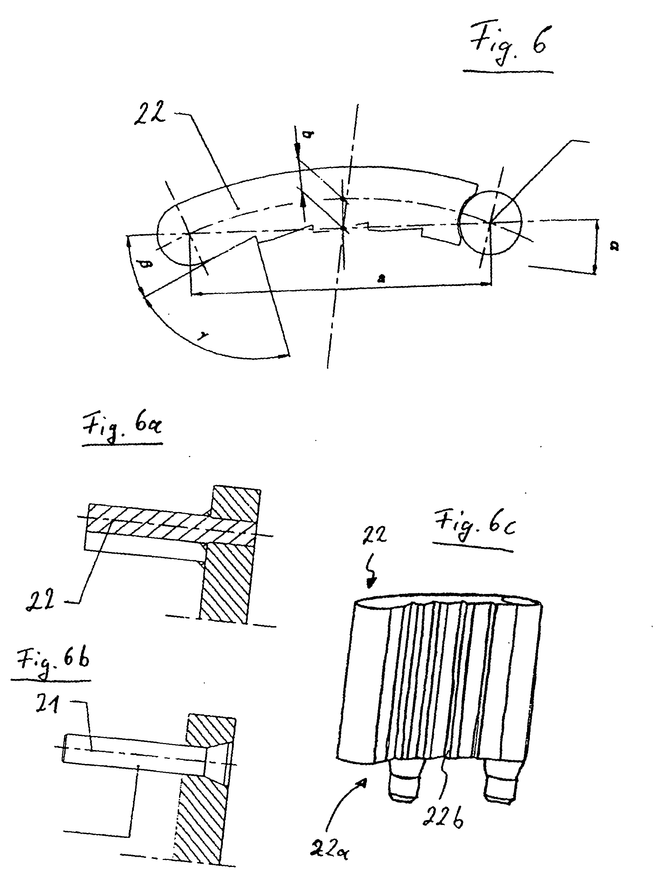

Die Figuren 6, 6a, 6b und 6c zeigen die Geometrie der Ventilatorschaufeln 22 und der Stifte 21. Der Kranz 20, die Ventilatorschaufeln 22 und die Stifte 21 sind aus Hartstahl gebaut. Eine Alternative ist, den Stift aus Porzellan und die Ventilatorschaufeln aus Stahl herzustellen. Die Neigung der Ventilatorschaufeln 22 beträgt im Verhältnis zur Horizontalen α = 4 - 15° mit der Optimierung auf 8 - 10°. Die Anordnung und die Größe von der Verzahnung ist von der Anzahl der Ventilatorschaufeln abhängig (β = 30 - 120° und γ = 60 - 120°). Der Biegungsgrad der Ventilatorschaufeln ist mit dem Verhältnis der Längen a und b definiert, wobei b 10 % der Sehnenlänge darstellt. Die Anordnung der Ventilatorschaufeln ist fixiert. Sie sind in die Kranzform in entsprechende Ausnehmungen eingepresst Die Form der Ventilatorschaufeln, profilierte Kollisionsoberfläche 22a, 22b und die Neigung sichern die Abfüllung der Ventilatorschaufeln mit dem Ausgangsmaterial und werden damit von der Abnutzungswirkung des Ausgangsmaterials geschützt, was auf die Verlängerung der Lebensdauer der Ventilatorschaufeln wirkt. Die Zähne 22b auf den Ventilatorschaufeln halten die erste Schicht des Ausgangsmaterials auf der Kollisionsoberfläche 22a, während die zweite Schicht langsam über die erste Schicht rutscht und diese zweite Schicht nimmt die Schläge des ankommenden Ausgangsmaterials an. Bevor die Ventilatorschaufeln 22 in die jeweilige Kranzreihe 20 der Rotorenscheibe 12 eingebracht wird, wird der Stift 21 in die Rotorenscheibe 12 eingepresst. Die Symmetrieachse des Stiftes 21 befindet sich in der Symmetrieachse des Biegungsgrades der Ventilatorschaufeln 22, damit die Geometrie des Systems der Ventilatorschaufein/Stifte optimal erreicht wird. Während der Materialverarbeitung schlagen die Materialkömchen auf die Stirnseite der Ventilatorschaufeln und insbesondere auf und in die Stifte. Der Stift wird somit abgenutzt. Nach der Abnutzung wird der Stift durch Auspressen oder Bohren durch einen neuen ausgetauscht. Dies hat einen entscheidenden Vorteil: Bisherige Varianten der Ventilatorschaufeln ohne Stifte haben die Abnutzung der Ventilatorschaufeln selbst ergeben. Der Austausch der abgenutzten Ventilatorschaufeln ist im Vergleich mit dem Austausch der Stifte sehr kompliziert, aufwendig und teuer. Die Stifte können daher leicht ausgetauscht werden; die Ventilatorschaufeln bleiben unbeschädigt und müssen es nicht. De Austausch der Ventilatorschaufeln erfolgt erst bei allgemeiner Materialermüdung.

Das Problem der Vibrationen und der Festigkeit der Stifte ist dadurch gelöst, dass sich der Stift 21 mit der Fläche von 1/3 seines Stiftumfanges an eine korrespondierende Ausnehmung der Ventilatorschaufel 22 an diese Ventilatorschaufeln "anlehnt", und sich nicht geradflächig an die Ventilatorschaufeln anlehnt. Die Ventilatorschaufeln 22 werden durch Kaltpressen verformt oder auch durch Schmieden, wobei das Schmieden der besseren Aushärtung dient.

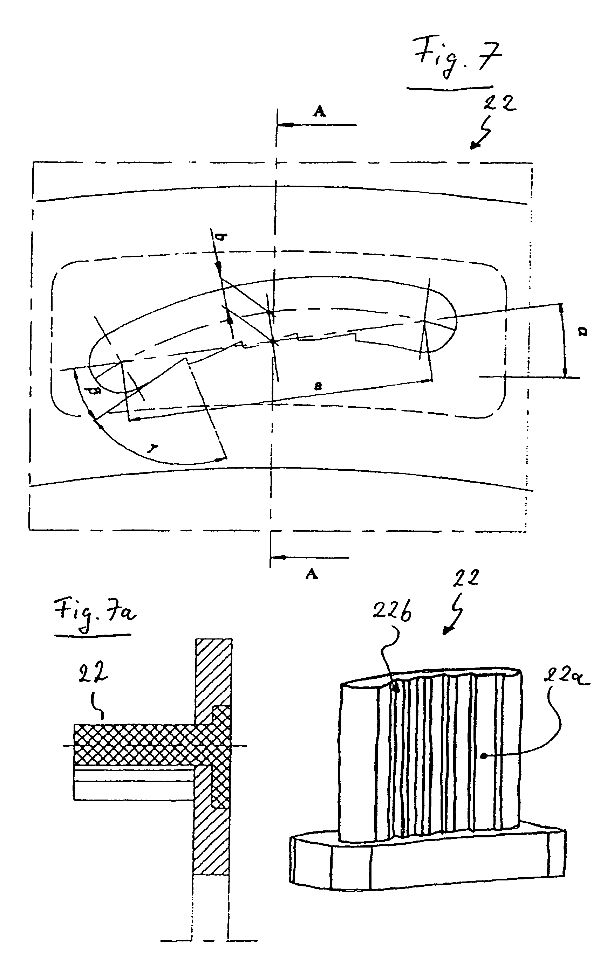

Eine andere Ausführungsform sieht erfindungsgemäß auch Segment-Ventilatorschaufeln (Figur 7, 7a) vor. Die Segment-Ventilatorschaufeln 22 werden aus Keramik oder Gussstahl hergestellt. Bei dieser Ausführungsform werden in die Rotorenscheiben 12 die Kanäle eingebaut, in welche die Segment-Ventilatorschaufeln mit unbestimmten Einlegungen gestellt werden. Die Genauigkeit der Einstellungen und die Festigkeit der Ventilatorschaufeln wird durch das Profil der Kanäle und durch die Reibung zwischen den Segment-Ventilatorschaufeln und die tragende Scheibe bestimmt. Die Neigung der Ventilatorschaufeln in Bezug auf die Horizontale beträgt α = 4 - 15° mit der Optimierung bei 8° - 10°. Die Einordnung der Zähne 22b und ihre Größe ist von der Länge der Ventilatorschaufeln 22 abhängig, Winkel β beträgt 30° - 120°, und Winkel γ beträgt 60° - 120°. Der Biegungsgrad der Ventilatorschaufeln ist mit dem Verhältnis der Längen a und b definiert, wobei b 10 % der Sehnenlänge darstellt. Die Form der Ventilatorschaufeln, der profilierten (verzahnten) Kollisionsoberfläche und der Neigung sichern, dass die Ventilatorschaufeln mit dem Ausgangsmaterial abgefüllt werden und damit von der Abnutzungswirkung des Ausgangsmaterials geschützt werden, was auf die Verlängerung der Lebensdauer der Ventilatorschaufeln wirkt. Die Zähne 22b auf den Ventilatorschaufeln halten wie in Figur 6 die erste Schicht des Ausgangsmaterials auf der Kollisionsoberfläche 22a, während die zweite Schicht langsam über die erste Schicht rutscht und diese zweite Schicht nimmt die Schläge des ankommenden Ausgangsmaterials an. Diese Ausführung hat im Vergleich zu obiger Ausführung (Fig. 6) den Vorteil, dass die Ventilatorschaufeln mit verschiedenen Neigungen (Winkel α) eingebaut werden können; somit ist der Austausch der Ventilatorschaufeln einfacher. Die Form der Ventilatorschaufein hat keine Aussparung für die Stifte.

### Bezugszeichen

- 10: Vorrichtung
- 11: Gehäuse
- 11a: Gehäuseseite zur Materialeinfuhr
- 11 b: Gehäuseseite mit Verschlussdeckel
- 11 c: Verschlussdeckel
- 11 d: Materialeinfuhrschacht
- 12: Rotorenscheiben
- 13: Motoren
- 13a: Riemen
- 14: Fundament / Gestell
- 15: Träger
- 16: Lager
- 17: Reckstangen
- 18: Einfuhrrohr
- 19: Auslass
- 20: Kränze
- 20a: Ausnehmung
- 21: Stifte
- 22: Ventilatorschaufeln
- 22a: Kollisionsfläche
- 22b: Verzahnung
- 23: Kanäle
- 24: Zermahlenes Material
- 25: Drehrichtung
- 26: Luftströme

## Patentansprüche

1. Verwendung von mikronisiertem Zeolith mit einem Korngrößendurchmesser von weniger als 0,5 µm, welcher durch Rotoren, die aus Scheiben bestehen, auf denen die Ventilatorschaufeln einseitig befestigt angeordnet sind und wobei die Ventilatorschaufeln mit den Kränzen verbunden sind und in korrespondierende Kanäle auf der jeweils gegenüberliegenden Rotorenscheibe durchgreifen, die einen Materialdurchgang unter den Ventilatorschaufeln verhindern, zerkleinert wurden, zur Herstellung eines pharmazeutischen Mittels.

2. Verwendung eines pharmazeutischen Mittels nach Anspruch 1 zur Herstellung eines pharmazeutischen Mittels zur Behandlung von Stoffwechsel-, sowie Herz-/Kreislauferkrankungen.

3. Verwendung eines pharmazeutischen Mittels nach Anspruch 1 zur Herstellung eines pharmazeutischen Mittels zur Behandlung von multipler Sklerose.

4. Verwendung eines pharmazeutischen Mittels nach Anspruch 1 zur Herstellung eines pharmazeutischen Mittels zur zur Behandlung von rheumatischen Erkrankungen.

5. Verwendung eines pharmazeutischen Mittels nach Anspruch 1 zur Herstellung eines pharmazeutischen Mittels zur Behandlung von dermatologischen Erkrankungen.

## Claims

1. Use of micronised zeolith with a grain diameter of less than 0,5 µm which is produced by rotors equipped with plates on which ventilator blades are attached on one side and in which the ventilator blades are connected to the rings and in corresponding channels of the appropriate rotor plate across from it which prevent the loss of material under the ventilator blades has been ground fine for the production of a pharmaceutical preparation.

2. Use of a pharmaceutical preparation according to claim 1 for the production of a pharmaceutical preparation for the treatment of metabolic as well as heart-/circulatory diseases.

3. Use of a pharmaceutical preparation according to claim 1 for the production of a pharmaceutical preparation for the treatment of multiple sclerosis.

4. Use of a pharmaceutical preparation according to claim 1 for the production of a pharmacutical preparation for the treatment of rheumatic diseases.

5. Use of a pharmaceutical preparation according to claim 1 for the production of a pharmaceutical preparation for the treatment of dermatological diseases.

## Revendications

1. Utilisation d'une zéolite micronisée de granulométrie inférieure à 0,5 µm pour préparer un agent pharmaceutique, qui a été broyée ou réduite en petits morceaux par des rotors constitués de disques sur lesquels sont fixées les pales de ventilateur disposées d'un seul côté, celles-ci étant raccordées aux couronnes et s'engrenant ou s'étendant dans des canaux correspondants sur le disque de rotor respectivement opposé qui empêchent le matériau de passer sous les pales de ventilateur.

2. Utilisation d'un agent pharmaceutique selon la revendication 1, pour la fabrication d'une préparation pharmaceutique destinée à traiter des affections ou pathologies du métabolisme, cardiaques et de la circulation.

3. Utilisation d'un agent pharmaceutique selon la revendication 1, pour la fabrication d'une préparation pharmaceutique destinée à traiter la sclérose en plaques.

4. Utilisation d'un agent pharmaceutique selon la revendication 1, pour la fabrication d'une préparation pharmaceutique destinée à traiter des affections ou pathologies rhumatismales.

5. Utilisation d'un agent pharmaceutique selon la revendication 1, pour la fabrication d'une préparation pharmaceutique destinée à traiter des affections ou pathologies dermatologiques.
